# EUROPÄISCHE PATENTSCHRIFT

(11) **EP 1 205 474 B1**
(45) Veröffentlichungstag und Bekanntmachung des Hinweises auf die Patenterteilung: **21.07.2004**
(21) Anmeldenummer: 01110421.3
(22) Anmeldetag: 27.04.2001
(51) Int. Cl.: C07D 301/12, C07D 303/02, B01J 23/16

(54) **Verfahren zur Epoxidation von cis-ständigen Doppelbindungen**
Epoxidation process of cis-double bonds
Procédé d'époxydation des doubles liaisons cis

(30) Priorität: 08.11.2000 DE 10055173
(43) Veröffentlichungstag der Anmeldung: 15.05.2002
(73) Patentinhaber: Degussa AG, 40474 Düsseldorf (DE)
(72) Erfinder: Schiffer, Thomas, Dr., 45721 Haltern (DE); Oenbrink, Georg, Dr., 48249 Dülmen (DE); Krebs, Bernt, Prof.Dr., 48163 Münster (DE); Droste, Elisabeth, Dr., 38642 Goslar (DE); Piepenbrink, Markus, 48159 Münster (DE); Vollmer, Guido, 33332 Gütersloh (DE)

(56) Entgegenhaltungen:
- EP-A- 0 549 077
- EP-A- 0 950 659
- GB-A- 2 055 821
- US-A- 3 806 467
- US-A- 4 562 276
- M. BÖSING ET AL.: "Highly Efficient Catalysts in Directed Oxygen-Transfer Processes: Synthesis, Sturctures of Novel Manganese-Containing Heteropolyanions, and Applications in Regioselective Epoxidation of Dienes with Hydrogen Peroxide" J. AM. CHEM. SOC., Bd. 120, Nr. 29, 1998, Seiten 7252-7259, XP002193323
- PIQUEMAL J-Y ET AL: "SYNTHESES, STRUCTURES ET REACTIVITE DE L'ANION (mu-HYDROGENOARSENATO)BIS(mu-PEROXO)BIS(OX OPEROXOTUNGSTATE)(2-) ET DE SON ANALOGUE AVEC LE LIGAND METHYLARSENATE CRISTALLISES AVEC LE CATION TETRABUTYLAMMONIUM" COMPTES RENDUS DE L ACADEMIE DES SCIENCES: SERIE II: MECANIQUE-PHYSIQUE-CHIMIE-ASTRONOMIE, EDITIONS SCIENTIFIQUES & MEDICALES ELSEVIER, FR, Bd. 319, Nr. 12, 15. Dezember 1994 (1994-12-15), Seiten 1481-1487, XP000494280 ISSN: 1251-8069
- SALLES L ET AL: "31P and 183W Spectroscopic Evidence for Novel Peroxo Species in the H3[PW12O40]yH2O System" INORGANIC CHEMISTRY, AMERICAN CHEMICAL SOCIETY, EASTON, US, Bd. 33, Nr. 5, 2. März 1994 (1994-03-02), Seiten 871-878, XP002078716 ISSN: 0020-1669

## Beschreibung

Die vorliegende Erfindung betrifft ein katalytisches Verfahren zur selektiven Epoxidation von cis-ständigen Doppelbindungen in makrocyclischen aliphatischen Kohlenwasserstoffen mit 8 bis 20 Ringkohlenstoffatomen und vorzugsweise 2 bis 5 isolierten Doppelbindungen, die gegebenenfalls eine oder mehrere Seitenketten tragen können, und in denen gleichzeitig mindestens eine weitere trans-ständige Doppelbindung anwesend ist.

Makrocyclische Olefine stellen wichtige Bausteine bei der Synthese zahlreicher Spezialchemikalien dar. Ein wichtiger Vertreter solcher cyclischer Kohlenwasserstoffe ist das cis,trans,trans-1,5,9-Cyclododecatrien, das großtechnisch durch Cyclotrimerisierung von 1,3-Butadien vorzugsweise an einem Titankatalysator gebildet wird und als Vorstufe für Cyclododecanon, Dodecandisäure und Laurinlactam / Polyamid 12 dient.

Zahlreiche Verfahren zur Epoxidation makrocyclischer Olefine sind bekannt. So lassen sich organische Persäuren wie Perameisensäure, Peressigsäure oder Perpropionsäure einsetzen. EP-A-0 033 763 (Degussa AG) beschreibt die Umsetzung von Cyclododecatrien (CDT) mit Perameisensäure, die in situ aus Wasserstoffperoxid und Ameisensäure gebildet wird. In EP-A-0 032 990 (Henkel KGaA, Degussa AG) werden Derivate des Cyclododecatriens mit Perameisensäure umgesetzt. Schwierigkeiten ergeben sich bei saureempfindlichen Olefinen aus Nebenreaktionen beziehungsweise Folgereaktionen, die die Epoxide unter den sauren Reaktionsbedingungen eingehen können. Die säurekatalysierte Addition von Wasser an das Epoxid führt zu einem vicinalen Diol; auch tritt Isomerisierung an den Doppelbindungen auf.

In EP-A-0 055 387 (Peroxid-Chemie) wird daher als Verbesserung nach der Synthese der Persaure eine Neutralisation der verbliebenen Carbonsäuren empfohlen, bevor epoxidiert wird. Neben Peressigsäure werden auch Perpropion- und Perbuttersäure als Oxidationsmittel beansprucht. Nachteilig bei diesem Verfahren sind die großen Mengen an Salz, die bei der Neutralisation anfallen. Die Oxidation mit anderen Persäuren wie meta-Chlorperbenzoesäure (M. D. E. Forbes, J. Phys. Chem. 97 (1993) 3390 - 3395) ist, wie bereits in EP-A-0 055 387 angemerkt wurde, im technischen Maßstab nicht wirtschaftlich.

Die Epoxidation mit Persäure verläuft bezüglich eines Angriffs an der cis-Doppelbindung unselektiv. W. Stumpf und K. Rombusch (Ann. Chem. 687 (1965) 136 - 149) konnten bei der systematischen Untersuchung der Umsetzung von cis,trans,trans-1,5,9-Cyclododecatrien mit Perameisensäure zeigen, dass zu 92 % eine der beiden trans-Doppelbindungen und nur zu 7 % die cis-Doppelbindung angegriffen wird. Daraus ergibt sich eine Reaktivität trans : cis von 6,5 : 1. Folglich werden bei einem Überschuss Oxidationsmittel bevorzugt beide trans-Doppelbindungen des cis,trans,trans-1,5,9-Cyclododecatriens angegriffen und es bilden sich erhebliche Mengen an Diepoxid.

Neben der unerwünschten trans-Selektivität ist beim Einsatz organischer Persäuren zu beachten, dass große Mengen an organischen Säuren anfallen, die vom Reaktionsgemisch abgetrennt und aufgearbeitet werden müssen. Zudem erfordert der Einsatz organischer Persäuren im technischen Maßstab einen erheblichen Sicherheitsaufwand, auf den auch in EP-A-0 032 990 und EP-A-0 033 763 hingewiesen wird. Bisher haben sich technische Verfahren mit organischen Persäuren zur Epoxidation von Cyclododecatrien nicht durchgesetzt.

Die Schwierigkeiten bei der Aufarbeitung großer Mengen organischer Carbonsäuren lassen sich umgehen, wenn anstelle der Persäure andere Sauerstoffquellen in Kombination mit katalytischen Mengen an Übergangsmetallsalz als Oxidationsmittel verwendet werden.

Die verwendeten Übergangsmetallkatalysatoren basieren häufig auf Verbindungen der 6. Nebengruppe des Periodensystems, insbesondere auf Wolfram und Molybdän. Bevorzugt werden diese Übergangsmetalle in Form ihrer Polysäuren oder Heteropolysäuren beziehungsweise als Polyoxometallat-Anionen eingesetzt.

Monoolefine lassen sich mit solchen Katalysatoren leicht durch einen großen Überschuss an Oxidationsmittel mit hohen Umsatzraten und Ausbeuten epoxidieren. Typische Monoolefine, an denen solche Katalysatorsysteme getestet werden, sind Cyclohexen oder Cycloocten. Als Oxidationsmittel finden neben Wasserstoffperoxid auch Luftsauerstoff (WO 98-54165, Yissum Research Institute; DD 212960, Akademie der Wissenschaften) oder Iodosylbenzol bzw. das pentafluorierte Derivat (US 4 864 041, Emory University) Verwendung.

In flüssiger Phase kann die Reaktion einphasig oder zweiphasig durchgeführt werden. Liegen zwei flüssige Phasen vor und wird das Übergangsmetall als homogener Katalysator verwendet, wird dessen Wirkung durch Phasentransferkatalysatoren, typischerweise quartäre Ammonium-, Pyridinium- oder Phosphoniumsalze, verbessert.

Verfahren, bei denen der Katalysator auf ein anorganisches oder organisches Trägermaterial aufgezogen ist, werden zum Beispiel in WO 93-00338 (Solvay Interox) und US 5 684 071 beschrieben.

Wesentlich schwieriger gestaltet sich die Synthese, wenn, wie beim Cyclododecatrien, selektiv nur eine von mehreren Doppelbindungen im Molekül epoxidiert werden soll. Beim derzeitigen Stand der Technik wird Wasserstoffperoxid im starken Unterschuss eingesetzt und die Reaktion muss nach relativ geringen Umsätzen (typischerweise < 25 %) abgebrochen werden, damit die Selektivität der Reaktion nicht unter 90% absinkt. Basierend auf diesem Stand der Technik wurde jüngst von Ube Industries in EP-A-0 950 659 ein technisches Verfahren zur Synthese von CDT-Monoepoxid beschrieben, bei dem eine mehrstufige Reaktorkaskade bei 20 °C bis 120 °C betrieben wird.

Als Katalysator wird von Ube die Kombination eines quartaren Ammoniumsalzes oder Pyridiniumsalzes mit einer wolframhaltigen Säure beziehungsweise deren Salzen, Dodecawolframat, wolframhaltigen Heteropolysäuren oder deren Salzen beansprucht. In den angegebenen Beispielen wird konkret die Umsetzung mit Natriumwolframat-Dihydrat (Na₂WO₄-2 H₂O) und Phosphorwolframsäure (H₃PO₄·12WO₃·x H₂O) beschrieben. Letztere hat den Nachteil, dass säureempfindliche Epoxide Folgereaktionen eingehen. Zudem wirkt die wässrige Lösung stark korrosiv.

In den Beispielen werden Cyclododecatrien und Wasserstoffperoxid im Verhältnis 4 : 1 eingesetzt. Am Ende der Reaktorkaskade sind zwischen 21,5 und 22,1 Mol-% CDT umgesetzt worden, wobei eine Selektivität an Monoepoxid zwischen 91,2 und 94,2 Mol-% erzielt wird.

Als Vergleich (EP-A-0 950 659, Beispiel 5) gibt Ube die Reaktion mit Phosphorwolframsäure im Labormaßstab bei einem Verhältnis CDT : H₂O₂ von 5 : 1 an, bei dem 18,2 Mol-% CDT mit einer Selektivität von 95,0 Mol-% zum Monoepoxid umgesetzt werden.

Die Patentanmelderin führt als Vorteil des eigenen Verfahrens die vergleichsweise hohe Umsatzrate und Ausbeute an. Letztere ist mit etwa 22 Mol-% zwar besser als im angegebenen Vergleichsbeispiel, dennoch müssen auch bei diesem Verfahren immer noch etwa 78 Mol-% unumgesetztes Cyclododecatrien in der Aufarbeitung abgetrennt und im Kreis geführt werden.

Aufgabe war es daher, ein Verfahren zu entwickeln, welches sich dadurch auszeichnet, dass eine cis-ständige Doppelbindung in einem makrocyclischen Olefin bei Anwesenheit mindestens einer weiteren trans-ständigen Doppelbindung bei einem Umsatz von größer als 25 % möglichst selektiv epoxidiert wird. Eine weitere Aufgabe der Erfindung bestand darin, ein neutrales Katalysatorsystem aufzufinden, um das Verfahren auch bei der Synthese säurelabiler Epoxide einsetzen zu können.

Überraschend wurde nun gefunden, dass beide Aufgaben dadurch gelöst werden, dass spezielle Polyoxometallate des Molybdäns und des Wolframs eingesetzt werden.

Gegenstand der Erfindung ist daher ein Verfahren zur Herstellung eines makrocyclischen Monoepoxides, welches noch mindestens eine Doppelbindung enthält, ausgehend von einem makrocyclischen aliphatischen Kohlenwasserstoff mit 8 bis 20 Ringkohlenstoffatomen, welcher mindestens eine cis-ständige Doppelbindung bei Anwesenheit von mindestens einer weiteren trans-ständigen Doppelbindung enthält, durch Einsatz von Wasserstoffperoxid in Gegenwart eines Katalysatorsystems, dadurch gekennzeichnet, dass in zwei flüssigen Phasen gearbeitet wird und dass ein homogenes Katalysatorsystem, bestehend aus einem Oxidationskatalysator und einem Phasentransferkatalysator, verwendet wird, wobei der Oxidationskatalysator aus einem Polyoxometallat des Wolframs oder des Molybdäns sowie aus mindestens einem weiteren Element der 14. bis 16. Gruppe des Periodensystems, ausgewählt aus der Gruppe, die aus den Elementen Germanium, Zinn, Blei, Arsen, Antimon, Bismut, Selen, Tellur und Polonium gebildet wird und optional ein oder mehrere Übergangsmetalle der 4. bis 12. Gruppe enthalt, und dass als Gegenion ein quartäres Ammoniumion, Pyridiniumion oder Phosphoniumion als Phasentransferkatalysator verwendet wird, besteht.

Typische Vertreter der Übergangsmetalle aus der 4. bis 12. Gruppe sind Titan, Vanadium, Chrom, Mangan, Eisen, Cobalt, Nickel, Palladium, Kupfer und Zink.

Erfindungsgemäße Vertreter der Polyoxometallat-Anionen besitzen bevorzugt Strukturen der Formel

[(M₂(AO₂)₂(XA₉O₃₃)₂ · y H₂O]ⁿ⁻,

[M₃(XA₉O₃₃)₂ · y H₂O]ⁿ⁻,

[M₄(XA₉O₃₄)₂ · y H₂O]ⁿ⁻,

oder

[M_{z}X(OH)_{3-z} (AO₃)₃ (XA₉O₃₃)]ⁿ⁻,

wobei die Buchstaben A, M und X für folgende Elemente stehen
M = Ti, V, Cr, Mn, Fe, Co, Ni, Pd, Cu, Zn, Sn, As
A = Mo, W
X = Ge, Sn, Pb, As, Sb, Bi, Se, Te, Po und
n eine Zahl von 6 bis 12,
y eine Zahl von 0 bis 6 und
z eine Zahl von 0 bis 3 ist.

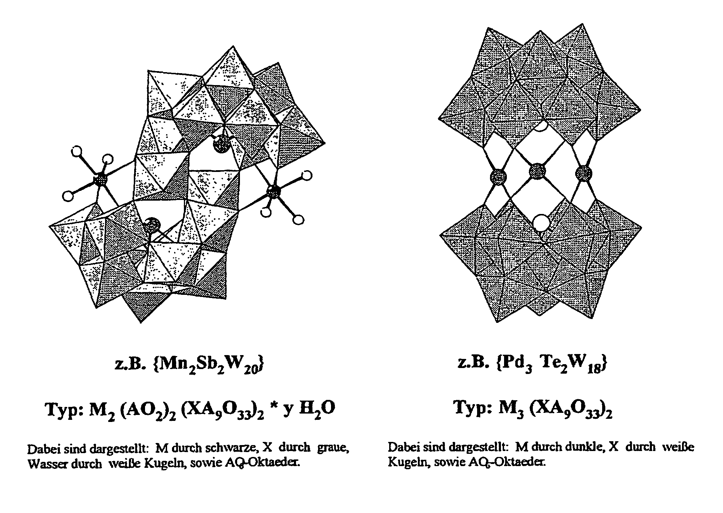

Als nicht limitierende Beispiele solcher Polyoxometallat-Anionen sind in Abbildung 1 die räumlichen Strukturen von M₂(AO₂)₂ (XA₉O₃₃)₂·6H₂O und M₃(MX₉O₃₃)₂ dargestellt, wie sie aus Röntgenstrukturanalysen bestimmt wurden.

Die Polyoxometallat-Anionen werden üblicherweise in verkürzter Schreibweise angegeben. Dabei werden die Sauerstoffatome, an den Heteroatomen gebundenes Wasser und die Ladung des Polyoxometallat-Anions weggelassen.

Die geschweiften Klammern zeigen an, dass es sich nicht um eine vollständige Summenformel, sondern um das Verhältnis der Elemente M, X und A handelt. So wird beispielsweise die Summenformel [(Mn₂ (WO₂)₂ (SbW₉O₃₃)₂·6 H₂O]¹⁰⁻ durch die verkürzte Schreibweise {Mn₂Sb₂W₂₀} wiedergegeben.

Die Polyoxometallate werden zunächst in Form ihrer Alkali- oder Erdalkalisalze, häufig als Natrium- oder Kaliumsalze, beziehungsweise ihrer Mischsalze hergestellt. Entsprechende Synthesen sind beispielsweise in der deutschen Patentanmeldung DE 195 30 787 A1, Inorg. Chem. 38 (1999), 2688 - 2694 oder Applied Catalysis A: General 184 (1999), 273 - 278, sowie der dort zitierten Literatur beschrieben.

Für die aktive Katalysatorspezies werden diese Kationen durch einen kationischen Phasen-Transfer-Katalysator ausgetauscht. Der aktive Katalysator ist sowohl in der wässrigen, als auch in der organischen Phase gut loslich und kann in der organischen Phase gelöst einige Tage bei 4 °C gelagert werden.

Typische Vertreter der verwendeten Kationen sind die von quartären Ammoniumsalzen mit üblichen Anionen der Form R₁R₂R₃R₄N⁺, wobei R₁ bis R₄ jeweils unabhängig voneinander für Wasserstoff oder eine Alkylgruppe mit 1 bis 20 Kohlenstoffatomen steht, die auch Verzweigungen enthalten darf. Nicht limitierende Beispiele für solche Kationen sind Methyltrioctylammonium, Cetyltrimethylammonium oder Tetrabutylammonium. Daneben können auch andere Phasen-Transfer-Kationen wie die von N-alkylierten Pyridiniumsalzen oder quartären Phosphoniumsalzen eingesetzt werden.

Die Umsetzung erfolgt in flüssiger Phase und kann sowohl diskontinuierlich zum Beispiel in einem Rührkessel als auch kontinuierlich zum Beispiel in einer Rührkesselkaskade erfolgen. Das System wird zweiphasig betrieben, wobei eine Phase aus einer wässrigen Lösung von Wasserstoffperoxid und die zweite Phase aus einem inerten, organischen, nicht mit Wasser mischbaren Lösemittel besteht. Nicht limitierende Beispiele für organische Lösemittel sind sowohl halogenhaltige Verbindungen wie z. B. Dichlormethan, 1,2-Dichlorethan, Chloroform und Tetrachlorkohlenstoff, als auch halogenfreie Verbindungen wie z. B. Benzol, Toluol, Xylole, oder Alkane und Cycloalkane mit insbesondere 5 bis 12 Kohlenstoffatomen wie z. B. n-Hexan.

Die Reaktion erfolgt üblicherweise zwischen 0 °C und 120 °C, bevorzugt zwischen 5 °C und 80 °C. Die Umsetzung erfolgt in der Regel bei Normaldruck; bei höheren Reaktionstemperaturen ergibt sich gegebenenfalls ein erhöhter Druck durch die Dampfdrücke der verwendeten Lösemittel.

Als Oxidationsmitel wird Wasserstoffperoxid verwendet. Lösungen mit einem Gehalt von 0,1 bis 85 % werden bevorzugt. Besonders bevorzugt werden 30 bis 70 %ige Lösungen. Das Lösemittel ist dabei vorzugsweise Wasser.

Umsatz und Selektivität werden gaschromatografisch verfolgt. Der Peroxidgehalt lässt sich durch Redoxtitration der wässrigen Phase bestimmen.

Der Abbruch der Reaktion erfolgt durch Separation der Phasen. Diese erfolgt bei diskontinuierlicher Fahrweise im Reaktor durch Abstellen des Rührers und Trennung der Phasen. Bei kontinuierlicher Fahrweise erfolgt die Phasentrennung üblicherweise in einem nachgeschalteten Trennbehälter.

Der Umsatz beträgt vorzugsweise mindestens 25 %.
Die Selektivität an Monoepoxid beträgt vorzugsweise mindestens 90 %. Im Besonderen beträgt der Anteil an trans,trans-5,9-Cyclododecadien-1-epoxid im Verhältnis zu cis,trans-5,9-Cyclododecadien-1-epoxid im Produktgemisch mindestens 35 Mol-%.

Die folgenden Beispiele sollen das Verfahren näher erläutern, ohne es dahingehend einzuschränken.

Für die Katalysatorsichtung (Beispiele 1 - 12 und die Vergleichsbeispiele 13 - 14) wurde der folgende Standardansatz gewählt. Dabei entsprechen die angegebenen Kurzformeln folgenden eingesetzten Polyoxometallat-Anionen:

{Mn₃Sb₂W₁₈} = [Mn₃ (SbW₉O₃₃)₂ · 3 H₂O]¹²⁻

{Mn₂Sb₂W₂₀} = [Mn₂ (WO₂)₂ (SbW₉O₃₃)₂ · 6 H₂O]¹⁰⁻

{Sn₂Sb₂W₂₀} = [Sn₂ (WO₂)₂ (SbW₉O₃₃)₂]¹⁰⁻

{Sn₂Bi₂W₂₀} = [Sn₂ (WO₂)₂ (BiW₉O₃₃)₂]¹⁰⁻

{Ni₂Te₂W₂₀} = [Ni₂ (WO₂)₂ (TeW₉O₃₃)₂ · 6 H₂O]⁸⁻

{Fe₂Sb₂W₂₀} = [Fe₂ (WO₂)₂ (SbW₉O₃₃)₂ · 6 H₂O]¹⁰⁻

{Mn₂Bi₂W₂₀} = [Mn₂ (WO₂)₂ (BiW₉O₃₃)₂ · 6 H₂O]¹⁰⁻

{V₂Bi₂W₂₀} = [V₂ (WO₂)₂ (BiW₉O₃₃)₂ · 6 H₂O]⁶⁻

{V₂Sb₂W₂₀} = [V₂ (WO₂)₂ (SbW₉O₃₃)₂ · 6 H₂O]⁶⁻

{Pd₄Te₂W₁₈} = [Pd₄ (TeW₉O₃₄)₂ · 2 H₂O]¹²⁻

{As₄Mo₁₂} = [(AsOH)₃(MoO₃)₃(AsMo₉O₃₃)]⁷⁻

{MnAs₄Mo₁₂} = [(AsOH)_{3-z} Mn_{z} (MoO₃)₃(AsMo₉O₃₃)]⁷⁻

### Herstellung der Katalysatorlösungen:

Zur Herstellung der Katalysatorlösungen werden 0,064 mmol des Polyoxometallates in Form des Alkali- bzw. Erdalkaliderivates mit 258 mg (0,64 mmol) Methyltrioctylammoniumchlorid in 20 ml eines 1 : 1-Gemisches von Wasser / 1,2-Dichlorethan durch Erwärmen unter Ruckfluss erhitzt, bis sich die Feststoffe gelöst haben und die wässrige Phase vollständig entfärbt hat (ca. 30 Minuten). Die farbige, organische Phase wird separiert und kann bei + 4 °C einige Tage gelagert werden.

### Epoxidation:

3,25 g (20,0 mmol) cis,trans,trans-1,5,9-Cyclododecatrien werden in 5 ml 1,2 Dichlorethan gelöst und mit 3,125 ml Katalysatorlösung (entspricht 0,02 mmol des jeweiligen Polyoxometallates) versetzt. Die Reaktionsmischung wurde mit 2,0 Äquivalenten. (40,0 mmol) Wasserstoffperoxid, eingesetzt als 30 %ige, wässrige Lösung, versetzt und bei Normaldruck und 25 °C mit 800 U/Min gerührt. Es ergibt sich somit ein Verhältnis Substrat / H₂O₂ /Katalysator von 1000 : 2000 : 1. Der Verlauf der Reaktion wird durch regelmäßige GC/MS-Messungen verfolgt.

Die Ergebnisse sind in folgenden Tabellen zusammengefasst.

### Beispiel 1:

| **{Mn**_{**3**}**Sb**_{**2**}**W**_{**18**}**}** | | | | | |
|---|---|---|---|---|---|
| Zeit [h] | Monoepoxid [%] | Diepoxid [%] | Umsatz CDT [%] | Selektivität [%] | TOF [mmol_{CDT}/(mmol _{Kat}·h)] |
| 1 | 24,3 | <0,1 | 24,3 | >99 | 243 |
| 2,5 | 42,0 | 4,2 | 46,2 | 90,9 | 185 |
| 4 | 51,9 | 7,2 | 60,1 | 86,4 | 150 |
| 6 | 56,1 | 15,4 | 71,5 | 78,5 | 119 |
| 30 | 38,3 | 58,9 | 97,2 | 39,4 | 41 |

### Beispiel 2:

| **{Mn**_{**2**}**Sb**_{**2**}**W**_{**20**}**}** | | | | | |
|---|---|---|---|---|---|
| Zeit [h] | Mono-Epoxid [%] | Di-Epoxid [%] | Umsatz CDT [%] | Selektivität [%] | TOF [mmol_{CDT}/(mmol _{Kat}·h)] |
| 1 | 12,1 | <0,1 | 12,1 | >99 | 121 |
| 4 | 38,9 | <0,1 | 38,9 | >99 | 97 |
| 6 | 52,9 | 6,9 | 59,8 | 88,5 | 99 |
| 24 | 55,4 | 36,1 | 91,5 | 60,5 | 38 |

### Beispiel 3:

| **{Sn**_{**2**}**Sb**_{**2**}**W**_{**20**}**}** | | | | | |
|---|---|---|---|---|---|
| Zeit [h] | Mono-Epoxid [%] | Di-Epoxid [%] | Umsatz CDT [%] | Selektivität [%] | TOF [mmol_{CDT}/(mmol _{Kat}·h)] |
| 1 | 12,4 | <0,1 | 12,4 | >99 | 124 |
| 4 | 51,3 | <0,1 | 51,3 | >99 | 128 |
| 6 | 66,2 | 1,5 | 67,7 | 97,8 | 113 |
| 24 | 48,7 | 45,4 | 94,1 | 51,8 | 39 |

### Beispiel 4:

| **{Sn**_{**2**}**Bi**_{**2**}**W**_{**20**}**}** | | | | | |
|---|---|---|---|---|---|
| Zeit [h] | Mono-Epoxid [%] | Di-Epoxid [%] | Umsatz CDT [%] | Selektivität [%] | TOF [mmol_{CDT}/(mmol _{Kat}·h)] |
| 1 | 9,0 | <0,1 | 9,0 | >99 | 90 |
| 4 | 57,4 | 0,8 | 58,2 | 98,2 | 146 |
| 6 | 57,9 | 11,1 | 69,0 | 83,9 | 115 |
| 24 | 53,8 | 38,8 | 92,6 | 58,1 | 39 |

### Beispiel 5:

| **{Ni**_{**2**}**Te**_{**2**}**W**_{**20**}**}** | | | | | |
|---|---|---|---|---|---|
| Zeit [h] | Mono-Epoxid [%] | Di-Epoxid [%] | Umsatz CDT [%] | Selektivität [%] | TOF [mmol_{CDT}/(mmol _{Kat}·h)] |
| 1 | 16,0 | 0,3 | 16,3 | 98,2 | 163 |
| 2 | 36,8 | 7,3 | 44,1 | 83,4 | 221 |
| 4 | 53,4 | 10,7 | 64,1 | 83,3 | 160 |
| 6 | 59,9 | 18,3 | 78,2 | 76,6 | 130 |
| 24 | 45,1 | 52,1 | 97,2 | 46,4 | 41 |

### Beispiel 6:

| **{Fe**_{**2**}**Sb**_{**2**}**W**_{**20**}**}** | | | | | |
|---|---|---|---|---|---|
| Zeit [h] | Mono-Epoxid [%] | Di-Epoxid [%] | Umsatz CDT [%] | Selektivität [%] | TOF [mmol_{CDT}/(mmol _{Kat}·h)] |
| 1 | 4,4 | <0,1 | 4,4 | >99 | 44 |
| 2 | 10,2 | <0,1 | 10,2 | >99 | 51 |
| 4 | 19,0 | <0,1 | 19,0 | >99 | 48 |
| 6 | 24,2 | 0,1 | 24,3 | >99 | 41 |
| 24 | 28,4 | 0,2 | 28,6 | >99 | 12 |

### Beispiel 7:

| **{Mn**_{**2**}**Bi**_{**2**}**W**_{**20**}**}** | | | | | |
|---|---|---|---|---|---|
| Zeit [h] | Mono-Epoxid [%] | Di-Epoxid [%] | Umsatz CDT [%] | Selektivität [%] | TOF [mmol_{CDT}/(mmol _{Kat}·h)] |
| 1 | 12,1 | <0,1 | 12,1 | >99 | 121 |
| 2 | 18,5 | <0,1 | 18,5 | >99 | 93 |
| 4 | 38,9 | 0,1 | 39,0 | >99 | 97 |
| 6 | 52,9 | 6,9 | 59,8 | 88,5 | 99 |
| 24 | 55,4 | 36,0 | 91,4 | 60,6 | 38 |

### Beispiel 8:

| **{V**_{**2**}**Bi**_{**2**}**W**_{**20**}**}** | | | | | |
|---|---|---|---|---|---|
| Zeit [h] | Mono-Epoxid [%] | Di-Epoxid [%] | Umsatz CDT [%] | Selektivität [%] | TOF [mmol_{CDT}/(mmol _{Kat}·h)] |
| 1 | 12,6 | <0,1 | 12,6 | >99 | 126 |
| 2 | 28,0 | <0,1 | 28,0 | >99 | 140 |
| 4 | 43,2 | 0,2 | 43,4 | >99 | 109 |
| 6 | 51,1 | 7,6 | 58,7 | 87,1 | 98 |
| 24 | 54,9 | 34,7 | 91,4 | 60,0 | 38 |

### Beispiel 9:

| **{V**_{**2**}**Sb**_{**2**}**W**_{**20**}**}** | | | | | |
|---|---|---|---|---|---|
| Zeit [h] | Mono-Epoxid [%] | Di-Epoxid [%] | Umsatz CDT [%] | Selektivität [%] | TOF [mmol_{CDT}/(mmol _{Kat}·h)] |
| 1 | 10,7 | <0,1 | 10,7 | >99 | 107 |
| 2 | 20,1 | 0,1 | 20,2 | >99 | 101 |
| 4 | 41,2 | 4,2 | 45,4 | 90,7 | 109 |
| 6 | 52,9 | 8,6 | 61,5 | 86,0 | 103 |
| 24 | 57,8 | 28,1 | 85,9 | 67,2 | 36 |

### Beispiel 10:

| **{Pd**_{**4**}**Te**_{**2**}**W**_{**18**}**}** | | | | | |
|---|---|---|---|---|---|
| Zeit [h] | Mono-Epoxid [%] | Di-Epoxid [%] | Umsatz CDT [%] | Selektivität [%] | TOF [mmol_{CDT}/(mmol_{Kat}·h)] |
| 1 | 13,4 | <0,1 | 13,4 | >99 | 134 |
| 2 | 24,9 | <0,1 | 24,9 | >99 | 125 |
| 4 | 41,3 | 0,2 | 41,5 | >99 | 104 |
| 6 | 50,3 | 6,7 | 57,0 | 88,2 | 95 |
| 24 | 55,2 | 22,9 | 78,1 | 70,7 | 33 |

### Beispiel 11:

| **{As**_{**4**}**Mo**_{**12**}**}** | | | | | |
|---|---|---|---|---|---|
| Zeit [h] | Mono-Epoxid [%] | Di-Epoxid [%] | Umsatz CDT [%] | Selektivität [%] | TOF [mmol_{CDT}/(mmol_{Kat}·h)] |
| 1 | 17,0 | <0,1 | 17,0 | >99 | 170 |
| 2 | 22,0 | <0,1 | 22,0 | >99 | 110 |
| 4 | 43,5 | 0,4 | 43,9 | 99,0 | 110 |
| 6 | 55,1 | 9,7 | 64,8 | 85,0 | 108 |
| 24 | 63,9 | 24,9 | 88,8 | 71,9 | 37 |

### Beispiel 12:

| **{(MnAs**_{**4**}**Mo**_{**12**}**}** | | | | | |
|---|---|---|---|---|---|
| Zeit [h] | Mono-Epoxid [%] | Di-Epoxid [%] | Umsatz CDT [%] | Selektivität [%] | TOF [mmol_{CDT}/(mmol _{Kat}·h)] |
| 1 | 7,0 | <0,1 | 7,0 | >99 | 70 |
| 2 | 10,1 | <0,1 | 10,1 | >99 | 50 |
| 4 | 18,1 | 0,1 | 18,2 | >99 | 46 |
| 6 | 26,3 | 0,2 | 26,5 | >99 | 44 |
| 24 | 54,0 | 7,9 | 63,9 | 84,5 | 27 |

Beispiele 13 - 16 zeigen Vergleichsversuche mit nicht erfindungsgemäßen Katalysatortypen. Beispiel 13 und 14 enthalten Polyoxometallate ohne ein Element der 14. - 16. Hauptgruppe, ausgewählt aus Germanium, Zinn, Blei, Arsen, Antimon, Bismut, Selen, Tellur und Polonium. Beispiele 15 und 16 verwenden anstelle der Polyoxometallat-Anionen eine Lösung aus Natriumwolframat Dihydrat, bzw. Phosphorwolframsäure (jeweils 0,02 mmol als wässrige Lösung mit Methyltrioctylammoniumchlorid als Phasen-Transfer-Katalysator) gemäß EP-A-0 950 659.

### Vergleichsbeispiel 13:

| **{Co**_{**5**}**W**_{**19**}**}** | | | | | |
|---|---|---|---|---|---|
| Zeit [h] | Mono-Epoxid [%] | Di-Epoxid [%] | Umsatz CDT [%] | Selektivität [%] | TOF [mmol_{CDT}/(mmol _{Kat}·h)] |
| 1 | <0,1 | 0,0 | <0,1 | - | 0 |
| 4 | 1,5 | <0,1 | 1,5 | >99 | 4 |
| 24 | 10,6 | 0,1 | 10,7 | 99,0 | 4 |

### Vergleichsbeispiel 14:

| **{Fe**_{**6**}**W**_{**18**}**}** | | | | | |
|---|---|---|---|---|---|
| Zeit [h] | Mono-Epoxid [%] | Di-Epoxid [%] | Umsatz CDT [%] | Selektivität [%] | TOF [mmol_{CDT}/(mmol _{Kat}·h)] |
| 24 | <0,1 | 0,0 | <0,1 | - | 0 |
| 75 | 0,6 | <0,1 | 0,6 | >99 | <1 |

### Vergleichsbeispiel 15:

| **Na**_{**2**}**WO**_{**4**}**·2 H**_{**2**}**O** | | | | | |
|---|---|---|---|---|---|
| Zeit [h] | Mono-Epoxid [%] | Di-Epoxid [%] | Umsatz CDT [%] | Selektivität [%] | TOF [mmol_{CDT}/(mmol _{Kat}·h)] |
| 24 | 0,0 | 0,0 | 0,0 | - | - |
| 75 | <0,1 | 0,0 | <0,1 | - | - |

### Vergleichsbeispiel 16:

| **H**_{**7**}**[PW**_{**12**}**O**_{**42**}**]** | | | | | |
|---|---|---|---|---|---|
| Zeit [h] | Mono-Epoxid [%] | Di-Epoxid [%] | Umsatz CDT [%] | Selektivität [%] | TOF [mmol_{CDT}/(mmol _{Kat}·h)] |
| 1 | 13,1 | <0,1 | 13,1 | >99 | 131 |
| 2 | 22,4 | 0,3 | 22,7 | 98,6 | 114 |
| 6,5 | 60,4 | 14,4 | 74,8 | 80,7 | 115 |
| 24* | 22,1 | 76,7 | 98,8 | 22,3 | 41 |

| | | | | | |
|---|---|---|---|---|---|
| * Durch säurekatalysierte Folgereaktionen bildeten sich bei Beispiel 10 nach einigen Stunden Nebenprodukte, zum Beispiel Cyclododecadien-1,2-diol durch Wasseranlagerung an das Epoxid. | | | | | |
| TOF = Turn Over Frequenz ("Umsatzgeschwindigkeit") | | | | | |

### Beispiel 17:

In einem 1 l-Reaktor mit Heizmantel und mechanischem Rührer (600 U/Min) werden 52 g (0,32 mol) cis,trans,trans-Cyclododecatrien in 400 ml Dichlormethan gelöst und mit 50 ml Katalysatorlösung gemäß Beispiel 2, entsprechend 0,32 mmol {Mn₂Sb₂W₂₀}, versetzt.

Die Reaktionsmischung wird auf 40 °C erwärmt. 31,2 g 35 gew.-%ige Peroxidlösung (0,32 mol Wasserstoffperoxid) werden innerhalb von 30 Minuten zudosiert und anschließend bei 40 °C weitere 60 Minuten gerührt. Nach einer Gesamtzeit von 90 Minuten beträgt der Umsatz an Cyclododecatrien 37,5 % bei einer Selektivität an Monoepoxid von 91,0 %.

### Beispiel 18:

Die Umsetzung erfolgt analog zu Beispiel 17. Es werden 20 ml Katalysatorlösung verwendet, dies entspricht einem Molverhältnis Edukt : Katalysator von 2500 : 1. Nach einer Reaktionszeit von 120 Minuten beträgt der Umsatz an Cyclododecatrien 34,0 % bei einer Selektivität an Monoepoxid von 92,2 %.

### Beispiel 19:

Die Umsetzung verläuft analog zu Beispiel 18, jedoch werden 20 ml Katalysator aus Beispiel 3 {Sn₂Sb₂W₂₀} und 400 ml Toluol als Lösemittel verwendet. Nach einer Reaktionszeit von 90 Minuten beträgt der Umsatz 34,2 % bei einer Selektivität an Monoepoxid von 90,1 %.

### Beispiel 20:

Die Umsetzung verläuft analog zu Beispiel 17, jedoch werden 50 ml Katalysatorlösung aus Beispiel 1 {Mn₃Sb₂W₁₈} und 400 ml Toluol als Lösemittel verwendet. 0,32 mol Wasserstoffperoxid werden kontinuierlich über eine Reaktionszeit von 6 Stunden dosiert. Nach 6 Stunden beträgt der Umsatz 34,2 % bei einer Selektivität an Monoepoxid von 91,7 %.

### Beispiel 21:

Die Umsetzung verläuft analog zu Beispiel 20, anstelle von Toluol wird o-Xylol eingesetzt. Es werden 50 ml Katalysatorlösung gemäß Beispiel 1, entsprechend 0,32 mmol {Mn₃Sb₂W₁₈} eingesetzt, allerdings wurde bei der Herstellung Methyltrioctylammoniumchlorid durch Tetrabutylphosphoniumchlorid ersetzt. Nach 6 Stunden betrug der Umsatz 30,6 % bei einer Selektivität an Monoepoxid von 91,8 %.

### Beispiel 22:

Die Umsetzung verläuft analog zu Beispiel 17. Es werden 50 ml Katalysatorlösung gemäß Beispiel 1, entsprechend 0,32 mmol {Mn₃Sb₂W₁₈} eingesetzt, allerdings wurde bei der Herstellung Methyltrioctylammoniumchlorid durch Tetrabutylphosphoniumchlorid ersetzt. Nach einer Reaktionszeit von 90 Minuten betrug der Umsatz 34,5 % bei einer Selektivität an Monoepoxid von 90,8 %.

### Beispiel 23: Bestimmung des Produktverhältnisses

Die Aufarbeitung erfolgt durch Trennung der Phasen, Trocknung der organischen Phase über Natriumsulfat und Filtration über Kieselgel zur Abtrennung des Katalysators. Das Lösemittel wird im Vakuum abgezogen, der Rückstand anschließend durch Rektifikation im Hochvakuum getrennt. Ausgehend von Beispiel 20 ließen sich 33,8 g CDT und 16,5 g Monoepoxide gewinnen, sowie ein hochsiedender Rückstand, der Diepoxide und Katalysatorreste enthielt. NMR-Analyse ergab 41,6 % trans,trans-5,9-Cyclododecadien-1-epoxid und 58, 4 % cis,trans-5,9-Cyclododecadien-1-epoxid. Dies entspricht einer Reaktivität cis : trans von 1,42 : 1.

## Patentansprüche

1. Verfahren zur Herstellung eines makrocyclischen Monoepoxids, welches noch mindestens eine Doppelbindung enthält, ausgehend von einem makrocyclischen aliphatischen Kohlenwasserstoff mit 8 bis 20 Ringkohlenstoffatomen, welcher mindestens eine cis-ständige Doppelbindung bei Anwesenheit von mindestens einer weiteren trans-ständigen Doppelbindung enthält, durch Einsatz von Wasserstoffperoxid in Gegenwart eines Katalysatorsystems,
. **dadurch gekennzeichnet, dass**
in zwei flussigen Phasen gearbeitet wird und dass ein homogenes Katalysatorsystem, bestehend aus einem Oxidationskatalysator und einem Phasentransferkatalysator, verwendet wird, wobei der Oxidationskatalysator aus einem Polyoxometallat des Wolframs oder des Molybdäns sowie aus mindestens einem weiteren Element der 14. bis 16. Gruppe des Periodensystems, ausgewählt aus der Gruppe, die aus den Elementen Germanium, Zinn, Blei, Arsen, Antimon, Wismut, Selen, Tellur und Polonium gebildet wird, besteht.

2. Verfahren nach Anspruch 1,
**dadurch gekennzeichnet,**
**dass** der eingesetzte makrocyclische Kohlenwasserstoff 2 bis 5 isolierte Doppelbindungen enthält.

3. Verfahren gemäß Anspruch 1,
**dadurch gekennzeichnet,**
**dass** eine cis-Doppelbindung im Makrocyclus epoxidert wird.

4. Verfahren gemäß Anspruch 1,
**dadurch gekennzeichnet,**
**dass** das Polyoxometallat-Anion zusätzlich ein oder mehrere Übergangsmetalle der 4. bis 12. Gruppe des Periodensystems enthält.

5. Verfahren gemäß Anspruch 4,
**dadurch gekennzeichnet,**
**dass** das zusätzliche Übergangsmetall ausgewählt wird aus der Gruppe der Elemente Titan, Vanadium, Chrom, Mangan, Eisen, Cobalt, Nickel, Palladium, Kupfer und Zink.

6. Verfahren gemäß einem der vorgehenden Ansprüche,
**dadurch gekennzeichnet,**
**dass** die Polyoxometallat-Anionen Strukturen der Formel
[(M₂(AO₂)₂ (XA₉O₃₃)₂ · y H₂O]ⁿ⁻,
[M₃(XA₉O₃₃)₂ · y H₂O]ⁿ⁻,
[M₄ (XA₉O₃₄)₂ · y H₂O]ⁿ⁻
oder
[M_{z}X(OH)_{3-z} (AO₃)₃ (XA₉O₃₃)]ⁿ⁻
besitzen,
wobei die Buchstaben A, M und X für
M = Ti, V, Cr, Mn, Fe, Co, Ni, Pd, Cu, Zn, Sn, As
A=Mo, W
X = Ge, Sn, Pb, As, Sb, Bi, Se, Te, Po stehen
und n eine Zahl von 6 bis 12,
y eine Zahl von 0 bis 6
und z eine Zahl von 0 bis 3 ist.

7. Verfahren gemäß einem der vorgehenden Ansprüche,
**dadurch gekennzeichnet,**
**dass** als Phasen-Transfer-Katalysator ein Kation verwendet wird, enthaltend in Verbindungen ausgewählt aus der Gruppe, die durch quartäre Ammoniumsalze, Pyridiumsalze und Phosphoniumsalze gebildet wird.

8. Verfahren gemäß einem der vorgehenden Ansprüche,
**dadurch gekennzeichnet,**
**dass** der Katalysator homogen in flüssiger Phase eingesetzt wird.

9. Verfahren gemäß einem der vorgehenden Ansprüche,
**dadurch gekennzeichnet,**
**dass** als Oxidationsmittel eine 0,1 bis 85 %ige wassrige Lösung von Wasserstoffperoxid verwendet wird.

10. Verfahren gemäß einem der vorgehenden Ansprüche,
**dadurch gekennzeichnet,**
**dass** ein inertes, organisches, nicht mit Wasser mischbares Lösemittel eingesetzt wird.

11. Verfahren gemäß Anspruch 10,
**dadurch gekennzeichnet,**
**dass** ein halogenhaltiges Lösemittel, ausgewählt aus der Gruppe, die durch Dichlormethan, 1,2-Dichorethan, Chloroform und Tetrachlorkohlenstoff gebildet wird, verwendet wird.

12. Verfahren gemäß Anspruch 10,
**dadurch gekennzeichnet,**
**dass** ein halogenfreies Lösemittel, ausgewählt aus der Gruppe, die durch Alkane, Cycloalkane, Benzol, Toluol und Xylole gebildet wird, verwendet wird.

13. Verfahren gemäß einem der vorgehenden Ansprüche,
**dadurch gekennzeichnet,**
**dass** die Reaktion bei 0 °C bis 120 °C durchgeführt wird.

14. Verfahren gemäß Anspruch 13,
**dadurch gekennzeichnet,**
**dass** die Reaktion bei 5 °C bis 80°C durchgeführt wird.

15. Verfahren gemäß einem der vorgehenden Ansprüche,
**dadurch gekennzeichnet,**
**dass** die Reaktion bei Normaldruck oder einem Druck stattfindet, der sich aus den Dampfdrücken der verwendeten Lösemittel unter den Reaktionsbedingungen ergibt.

16. Verfahren gemäß einem der vorherigen Ansprüche,
**dadurch gekennzeichnet,**
**dass** als Edukt cis,trans,trans-1,5,9-Cyclododecatrien eingesetzt wird.

17. Verfahren gemäß Anspruch 16,
**dadurch gekennzeichnet,**
**dass** der Umsatz mindestens 25 % beträgt.

18. Verfahren gemäß Anspruch 17,
**dadurch gekennzeichnet,**
**dass** die Selektivität an Monoepoxid über 90 % beträgt.

19. Verfahren gemäß Anspruch 16,
**dadurch gekennzeichnet,**
**dass** der Anteil an trans, trans-5,9-Cyclododecadien-1-epoxid im Verhältnis zu cis, trans-5,9-Cyclododecadien-1-epoxid im Produktgemisch mindestens 35 Mol-% beträgt.

## Claims

1. A process for the preparation of a macrocyclic monoepoxide which also contains at least one double bond, starting from a macrocyclic aliphatic hydrocarbon having 8 to 20 ring carbon atoms which contains at least one cis double bond in the presence of at least one further trans double bond, by using hydrogen peroxide in the presence of a catalyst system, **characterized in that** it comprises working in two liquid phases and using a homogeneous catalyst system consisting of an oxidation catalyst and a phase-transfer catalyst, where the oxidation catalyst consists of a polyoxometallate of tungsten or of molybdenum and at least one further element from group 14 to 16 of the Periodic Table, selected from the group formed by the elements germanium, tin, lead, arsenic, antimony, bismuth, selenium, tellurium and polonium.

2. A process according to claim 1, **characterized in that** the macrocyclic hydrocarbon employed contains 2 to 5 isolated double bonds.

3. A process according to claim 1, **characterized in that** a cis double bond in the macrocycle is epoxidized.

4. A process according to claim 1, **characterized in that** the polyoxometallate anion additionally contains one or more transition metals from group 4 to 12 of the Periodic Table.

5. A process according to claim 4, **characterized in that** the additional transition metal is selected from the group consisting of the elements titanium, vanadium, chromium, manganese, iron, cobalt, nickel, palladium, copper and zinc.

6. A process according to any one of the preceding claims, **characterized in that** the polyoxometallate anions have structures of the formula
[ (M₂(AO₂)₂(XA₉O₃₃)₂·y H₂O]ⁿ⁻,
[M₃(XA₉O₃₃)₂·y H₂O]ⁿ⁻,
[M₄(XA₉O₃₄)₂·y H₂O]ⁿ⁻,
or
[M_{z}X(OH)_{3-z}(AO₃)₃(XA₉O₃₃)]ⁿ⁻,
where the letters A, M and X represent the following elements:
M = Ti, V, Cr, Mn, Fe, Co, Ni, Pd, Cu, Zn, Sn or As
A = Mo or W
X = Ge, Sn, Pb, As, Sb, Bi, Se, Te or Po,
and n is a number from 6 to 12,
y is a number from 0 to 6, and
z is a number from 0 to 3.

7. A process according to any one of the preceding claims, **characterized in that** the phase-transfer catalyst used is a cation present in compounds selected from the group formed by quaternary ammonium salts, pyridinium salts and phosphonium salts.

8. A process according to any one of the preceding claims, **characterized in that** the catalyst is employed in homogeneous form in the liquid phase.

9. A process according to any one of the preceding claims, **characterized in that** the oxidant used is a 0.1 to 85% strength aqueous solution of hydrogen peroxide.

10. A process according to any one of the preceding claims, **characterized in that** an inert, organic, water-immiscible solvent is employed.

11. A process according to claim 10, **characterized in that** a halogen-containing solvent selected from the group formed by dichloromethane, 1,2-dichloroethane, chloroform and tetrachloromethane is used.

12. A process according to claim 10, **characterized in that** a halogen-free solvent selected from the group formed by alkanes, cycloalkanes, benzene, toluene and xylenes is used.

13. A process according to any one of the preceding claims, **characterized in that** the reaction is carried out at from 0°C to 120°C.

14. A process according to claim 13, **characterized in that** the reaction is carried out at from 5°C to 80°C.

15. A process according to any one of the preceding claims, **characterized in that** the reaction is carried out at atmospheric pressure or at a pressure which arises from the vapour pressures of the solvents used under the reaction conditions.

16. A process according to any one of the preceding claims, **characterized in that** the starting material employed is cis,trans,trans-1,5,9-cyclododecatriene.

17. A process according to claim 16, **characterized in that** the conversion is at least 25%.

18. A process according to claim 17, **characterized in that** the monoepoxide selectivity is greater than 90%.

19. A process according to claim 16, **characterized in that** the proportion of trans,trans-5,9-cyclododecadiene 1-epoxide relative to cis,trans-5,9-cyclododecadiene 1-epoxide in the product mixture is at least 35 mol%.

## Revendications

1. Procédé pour la fabrication d'un monoépoxyde macrocyclique qui contient également au moins une double liaison à partir d'un hydrocarbure aliphatique macrocyclique ayant 8 à 20 atomes de carbone, lequel contient au moins une double liaison cis en présence d'au moins une autre double liaison trans, en utilisant du peroxyde d'hydrogène en présence d'un système catalytique,
**caractérisé en ce qu'**
on travaille en deux phases liquides et un système catalytique homogène se composant d'un catalyseur d'oxydation et d'un catalyseur de transfert de phase est utilisé, le catalyseur d'oxydation se composant d'un polyoxométallate de tungstène ou de molybdène, ainsi que d'au moins un autre élément du 14^{ième} au 16^{ième} groupe du tableau périodique, choisi dans le groupe formé par les éléments germanium, étain, plomb, arsène, antimoine, bismuth, sélènium, tellure et polonium.

2. Procédé selon la revendication 1,
**caractérisé en ce que**
l'hydrocarbure macrocyclique utilisé contient 2 à 5 doubles liaisons isolées.

3. Procédé selon la revendication 1,
**caractérisé en ce qu'**
une double liaison cis est époxydée dans un macrocycle.

4. Procédé selon la revendication 1,
**caractérisé en ce que**
l'anion polyoxométallate comprend en outre un ou plusieurs métaux de transition, du 4^{ième} au 12^{ième} groupe du tableau périodique.

5. Procédé selon la revendication 4,
**caractérisé en ce que**
le métal de transition complémentaire est sélectionné dans les groupes des éléments titane, vanadium, chrome, manganèse, fer, cobalt, nickel, palladium, cuivre et zinc.

6. Procédé selon l'une quelconque des revendications précédentes,
**caractérisé en ce que**
les anions polyoxométallates possèdent des structures de formule
[(M₂(AO₂)₂(XA₉O₃₃)₂· yH₂O]ⁿ⁻,
[M₃(XA₉O₃₃)₂·yH₂O]ⁿ⁻,
[M₄(XA₉O₃₄)2·yH₂O]ⁿ⁻,
ou
[M_{z}X(OH)_{3-z}(AO₃)₃(XA₉O₃₃)]ⁿ⁻,
dans lesquelles les lettres A, M et X désignent les éléments suivants :
M = Ti, V, Cr, Mn, Fe, Co, Ni, Pd, Cu, Zn, Sn, As
A = Mo, W
X = Ge, Sn, Pb, As, Sb, Bi, Se, Te, Po et
n représente un nombre de 6 à 12,
y représente un nombre de 0 à 6 et
z représente un nombre de 0 à 3.

7. Procédé selon l'une quelconque des revendications précédentes,
**caractérisé en ce que**
comme catalyseur de transfert de phase, on utilise un cation qui contient des composés choisis dans le groupe constitué de sels d'ammonium, de sels de pyridinium et de sels de phosphonium quaternaires.

8. Procédé selon l'une quelconque des revendications précédentes,
**caractérisé en ce que**
le catalyseur est utilisé de façon homogène dans une phase liquide.

9. Procédé selon l'une quelconque des revendications précédentes,
**caractérisé en ce que**
comme agent d'oxydation, on utilise une solution aqueuse de 0,1 à 85 % de peroxyde d'hydrogène.

10. Procédé selon l'une quelconque des revendications précédentes,
**caractérisé en ce qu'**
on utilise un solvant organique et inerte non miscible dans l'eau.

11. Procédé selon la revendication 10,
**caractérisé en ce qu'**
on utilise un solvant contenant de l'halogène et choisi dans le groupe constitué de dichlorométhane, de 1,2-dichloroéthane, de chloroforme et de tétrachlorocarbone.

12. Procédé selon la revendication 10,
**caractérisé en ce qu'**
on utilise un solvant exempt d'halogène choisi dans le groupe constitué des alcanes, des cycloalcanes, du benzène, du toluène et des xylènes.

13. Procédé selon l'une quelconque des revendications précédentes,
**caractérisé en ce que**
la réaction est réalisée entre 0°C et 120°C.

14. Procédé selon la revendication 13,
**caractérisé en ce que**
la réaction est réalisée entre 5°C et 80°C.

15. Procédé selon l'une quelconque des revendications précédentes,
**caractérisé en ce que**
la réaction a lieu à une pression normale ou à une pression qui résulte des pressions de condensation des solvants utilisés dans les conditions de réaction.

16. Procédé selon l'une quelconque des revendications précédentes,
**caractérisé en ce que**
comme produit d'addition on utilise du cis,trans,trans-1,5,9-cyclododécatriène.

17. Procédé selon la revendication 16,
**caractérisé en ce que**
le rendement s'élève au moins à 25 %.

18. Procédé selon la revendication 17,
**caractérisé en ce que**
la sélectivité en monoépoxyde s'élève à plus de 90 %.

19. Procédé selon la revendication 16,
**caractérisé en ce que**
la proportion en trans,trans-5,9-cyclododécadièn-1-époxyde par rapport à cis,trans-5,9-cyclododécadièn-1-époxyde dans un mélange de produits s'élève à au moins 35 % en moles.
